# EUROPEAN PATENT APPLICATION

(11) **EP 4 331 580 A1**
(43) Date of publication of application: **06.03.2024**
(21) Application number: 22795405.4
(22) Date of filing: 25.03.2022
(51) Int. Cl.: A61K 31/4172, A23L 33/175, A61P 3/00, A61P 3/02, A61P 7/00, A61P 7/06, A61P 43/00

(54) **COMPOSITION FOR INCREASING RED BLOOD CELLS AND/OR HEMOGLOBIN**

(30) Priority: 26.04.2021 JP 2021074320
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); MURAYAMA, Norihito, Soraku-gun, Kyoto 619-0284 (JP)
(74) Representative: Vossius & Partner Patentanwälte Rechtsanwälte mbB
(86) International application number: PCT/JP2022/014221
(87) International publication number: WO 2022/230487

(57) **Abstract**

The present invention aims to provide a composition for increasing at least one of red blood cell count or hemoglobin level, a composition for preventing or reducing anemia, a composition for improving endurance, an antifatigue composition, a method of increasing at least one of red blood cell count or hemoglobin level, a method of preventing or reducing anemia, a method of improving endurance, and a method of reducing, alleviating, or preventing fatigue or recovering from fatigue. The present invention relates to a composition for increasing at least one of red blood cell count or hemoglobin level, containing L-ergothioneine or a salt thereof as an active ingredient.

## Description

### TECHNICAL FIELD

The present invention relates to a composition for increasing at least one of red blood cell count or hemoglobin level. The present invention also relates to a composition for preventing or reducing anemia, a composition for improving endurance, and an antifatigue composition. The present invention further relates to, for example, a method of increasing at least one of red blood cell count or hemoglobin level, a method of preventing or reducing anemia, a method of improving endurance, and a method of reducing, alleviating, or preventing fatigue or recovering from fatigue.

### BACKGROUND ART

Red blood cells are a type of blood cells and flow throughout the body through blood circulation. Red blood cells play a role in carrying oxygen from the lungs to the cells in every corner of the body. Red blood cells also remove carbon dioxide. Red blood cells are filled with hemoglobin, which is a red protein containing iron. Oxygen is taken into the hemoglobin in red blood cells. In other words, red blood cells and hemoglobin function to carry oxygen in the body. An increase in red blood cell count in the blood and blood hemoglobin level are known to improve the capacity to carry oxygen in the blood (oxygen-carrying capacity) and lead to an improvement in endurance.

A decrease in red blood cell count in the blood and blood hemoglobin level is known to cause anemia.
Generally, progression of anemia reduces the oxygen-carrying capacity due to decreased complete blood count in the blood, especially decreased hemoglobin level, resulting in tissue oxygen deprivation. This causes symptoms such as shortness of breath after exercise, palpitations, dizziness, lightheadedness, ringing in the ears, head congestion or headache, appetite loss, constipation, diarrhea, feelings of fatigue, malaise, cold hands and feet, cardiac palpitations, irregular menstruation, abnormal urination, decreased libido, dyspnea at rest, syncope, and angina pectoris. Even mild anemia can cause such symptoms described above. Anemia is a problem to many people regardless of gender and age. Thus, studies have been conducted on components effective in preventing or reducing anemia, having an action to increase the red blood cell count and/or hemoglobin level, and applicable to foods for specified health uses, foods with function claims, pharmaceutical products, and the like.

Ergothioneine is an amino acid found in mushrooms and the like. L-ergothioneine is present in nature. According to Patent Literature 1, the peritesticular fat weight and the triglyceride (neutral fat) level in plasma were lower in mice orally administered with ergothioneine than in mice not orally administered with ergothioneine.

### CITATION LIST

### - Patent Literature

Patent Literature 1: JP 2011-102286 A

### SUMMARY OF INVENTION

### - Technical Problem

However, Patent Literature 1 is silent about an action of L-ergothioneine to increase the red blood cell count and/or hemoglobin level.

The present invention aims to provide a composition for increasing at least one of red blood cell count or hemoglobin level. The present invention also aims to provide a composition for preventing or reducing anemia, a composition for improving endurance, and an antifatigue composition. The present invention also aims to provide a method of increasing at least one of red blood cell count or hemoglobin level, a method of preventing or reducing anemia, method of improving endurance, and a method of reducing, alleviating, or preventing fatigue or recovering from fatigue.

### - Solution to Problem

As a result of extensive studies to solve the problem, the present inventors found that L-ergothioneine has an action to increase the red blood cell count and/or hemoglobin level.

Specifically, the present invention relates to, but is not limited to, a composition for increasing at least one of red blood cell count or hemoglobin level, a method of increasing at least one of red blood cell count or hemoglobin level, and the like described below.
(1) A composition for increasing at least one of red blood cell count or hemoglobin level, containing L-ergothioneine or a salt thereof as an active ingredient.
(2) A composition for preventing or reducing anemia, containing the composition according to (1) above.
(3) A composition for improving endurance, containing the composition according to (1) above.
(4) An antifatigue composition, containing the composition according to (1) above.
(5) The composition according to any one of (1) to (4) above for increasing blood taurine level.
(6) The composition according to any one of (1) to (5) above, wherein the composition is an oral composition.
(7) The composition according to any one of (1) to (6) above, wherein the composition is a food or beverage.
(8) The composition according to any one of (1) to (7) above, wherein an amount of L-ergothioneine or a salt in the composition per adult daily intake is 2 to 50 mg in terms of L-ergothioneine.
(9) A method of increasing at least one of red blood cell count or hemoglobin level, including administering L-ergothioneine or a salt thereof.
(10) A method of preventing or reducing anemia, including administering L-ergothioneine or a salt thereof.
(11) A method of improving endurance, including administering L-ergothioneine or a salt thereof.
(12) A method of reducing, alleviating, or preventing fatigue or recovering from fatigue, including administering L-ergothioneine or a salt thereof.
(13) Use of L-ergothioneine or a salt thereof for increasing at least one of red blood cell count or hemoglobin level.
(14) Use of L-ergothioneine or a salt thereof for preventing or reducing anemia.
(15) Use of L-ergothioneine or a salt thereof for improving endurance.
(16) Use of L-ergothioneine or a salt thereof for reducing, alleviating, or preventing fatigue or recovering from fatigue.

### - Advantageous Effects of Invention

The present invention can provide a composition for increasing at least one of red blood cell count or hemoglobin level. The present invention can provide a composition for preventing or reducing anemia, a composition for improving endurance, and an antifatigue composition. The present invention can also provide a method of increasing at least one of red blood cell count or hemoglobin level, a method of preventing or reducing anemia, a method of improving endurance, and a method of reducing, alleviating, or preventing fatigue or recovering from fatigue.

### BRIEF DESCRIPTION OF DRAWINGS

FIG. 1 is a graph showing change in red blood cell count in the blood of a test food group and a control food group between pre-testing and testing on week 4.
FIG. 2 is a graph showing change in blood hemoglobin concentration of the test food group and the control food group between pre-testing and testing on week 4.
FIG. 3 is a graph showing change in blood taurine level of the test food group and the control food group between pre-testing and testing on week 4.

### DESCRIPTION OF EMBODIMENTS

The composition for increasing at least one of red blood cell count or hemoglobin level of the present invention contains L-ergothioneine or a salt thereof as an active ingredient. Hereinafter, the composition for increasing at least one of red blood cell count or hemoglobin level of the present invention is also referred to as "the composition according to the first embodiment of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula edodes*), hen-of-the-wood (binomial name: *Grifola Frondosa*), reishi mushuroom (binomial name: *Ganoderma lucidum*), lion's mane mushroom (binomial name: *Hericium erinaceus*), Yanagi-matsutake (binomial name: *Agrocybe aegerita*), girolle (binomial name: *Cantharellus cibarius*)*,* porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

As described later in Examples, the red blood cell count in the blood and blood hemoglobin concentration were significantly increased when L-ergothioneine was ingested compared to when no L-ergothioneine was ingested.

Thus, L-ergothioneine can be used as an active ingredient to promote an increase in red blood cell count in the blood and/or blood hemoglobin level.

L-ergothioneine or a salt thereof has action to increase the red blood cell count and blood hemoglobin level and thus has an effect of preventing or reducing anemia.

The action to increase the red blood cell count in the blood may be provided by reducing or suppressing a decrease in red blood cell count, promoting the recovery of red blood cell count, or any other way. The action refers to the achievement of an effect of increasing the red blood cell count in the blood by ingestion of L-ergothioneine or a salt thereof.

The action to increase the blood hemoglobin level may be provided by reducing or suppressing a decrease in blood hemoglobin level, promoting an increase in blood hemoglobin level, or any other way. The action refers to achievement of an effect of increasing the blood hemoglobin concentration by ingestion of L-ergothioneine or a salt thereof.

L-ergothioneine or a salt thereof has an action to increase the red blood cell count in the blood and/or blood hemoglobin level and thus can be used as an active ingredient for, for example, preventing or reducing anemia, improving endurance, or preventing or reducing fatigue. The present invention also encompasses a composition for preventing or reducing anemia, a composition for improving endurance, and an antifatigue composition, which contain L-ergothioneine or a salt thereof as an active ingredient. In one embodiment, the composition according to the first embodiment of the present invention can be used to prevent or reduce anemia or to improve endurance.

The composition according to the first embodiment of the present invention can be used in a composition for preventing or reducing anemia, a composition for improving endurance, or an antifatigue composition.

The present invention also encompasses a composition for preventing or reducing anemia, which contains the composition according to the first embodiment of the present invention. The composition for preventing or reducing anemia of the present invention (also referred to as "the composition according to the second embodiment of the present invention") has an action to prevent anemia or an action to reduced anemia. The composition for preventing or reducing anemia of the present invention can be used to prevent or ameliorate a condition or disease caused by anemia.

Preventing a condition or disease encompasses preventing the onset of a condition or disease, delaying the onset of a condition or disease, reducing the incidence of a condition or disease, reducing the onset risk of a condition or disease, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

Examples of the condition or disease caused by anemia include shortness of breath, palpitations, dizziness, lightheadedness, ringing in the ears, head congestion or headache, appetite loss, constipation, diarrhea, feelings of fatigue, malaise, cold hands and feet, cardiac palpitations, irregular menstruation, abnormal urination, decreased libido, dyspnea at rest, syncope, and angina pectoris

The present invention also encompasses a composition for improving endurance, which contains the composition according to the first embodiment of the present invention. The composition according to the first embodiment of the present invention has an action to increase the red blood cell count in the blood and/or blood hemoglobin level. Thus, the composition for improving endurance can improve or enhance the blood oxygen-carrying capacity and provide an action to improve endurance. The composition for improving endurance of the present invention (also referred to as "the composition according to the third embodiment of the present invention") also encompasses concepts of a composition for preventing a decrease in endurance and a composition for maintaining endurance.

The present invention also encompasses an antifatigue composition containing the composition according to the first embodiment of the present invention (also referred to as "the composition according to the fourth embodiment of the present invention"). The composition according to the first embodiment of the present invention has an action to increase the red blood cell count in the blood and/or blood hemoglobin level. Thus, the antifatigue composition can improve or enhance the blood oxygen-carrying capacity and provide an antifatigue action.

Fatigue is a phenomenon in which the physical and mental performance of a subject is temporarily reduced when the subject is under continuous physical or mental stress. The reduction in performance means a qualitative or quantitative reduction in physical and mental work capacity.

In the present invention, the antifatigue action refers to an action to reduce the fatigue, an action to alleviate the fatigue, an action to recover from the fatigue, and an action to prevent susceptibility to the fatigue (e.g., an action to prevent the fatigue). Specifically, the antifatigue action refers to an effect of promoting an improvement in working duration of a part (including the brain) used for exercise or action while preventing an increase in fatigue factors from the same amount of exercise or action (improving endurance and enhancing physical fitness); an effect of promoting an improvement in the state of brain and nerves that are sensing fatigue of a part used for exercise or action although the part is not actually fatigued; and an effect of promoting recovery of the fatigue state of a part used for exercise or action back to the normal state; and the like. In one embodiment, the composition according to the first embodiment of the present invention can be used, for example, for reducing, alleviating, or preventing fatigue or recovering from fatigue.

When feeling physical fatigue during muscle exercise such as sports or feeling mental fatigue during continuous work such as calculation, ingestion of the antifatigue composition of the present invention can help recover from fatigue. In addition, ingestion of the antifatigue composition of the present invention before work or sports can prevent fatigue from the work or sports. Ingestion before or during sports is likely to result in improved endurance. Further, daily ingestion can prevent fatigue and a disease associated with fatigue and can promote recovery from fatigue on a daily basis.

The antifatigue action, i.e., the effect of the "antifatigue composition" can be confirmed not only by measuring the red blood cell count in the blood and/or blood hemoglobin concentration but also by measuring the blood taurine concentration. Human studies have confirmed that taurine administration produces an effect of helping remove waste products that cause muscle fatigue and wear and reducing muscle cell damage and oxidative stress. Thus, when the blood taurine concentration is increased, the effect of reducing muscle cell damage is observed, and a reduction in feelings of physical fatigue is observed. When the antifatigue composition of the present invention is ingested in advance, and an increase in red blood cell count in the blood and/or blood hemoglobin level as well an increase in taurine level are observed, an antifatigue effect is observed.

Herein, the composition for increasing at least one of red blood cell count or hemoglobin level of the present invention, the composition for preventing or reducing anemia, the composition for improving endurance, and the antifatigue composition may be simply collectively referred to as "the composition of the present invention" in the following description.

Preferably, the composition of the present invention is a composition for increasing the blood taurine level. Taurine is a type of amino acids.

As described later in Examples, the blood taurine concentration was significantly increased when L-ergothioneine was ingested compared to when no L-ergothioneine was ingested.

Thus, the composition of the present invention containing L-ergothioneine or a salt thereof can be used to increase the blood taurine level.

The composition of the present invention has an action to increase the blood taurine level and thus can be used to obtain benefits from increased taurine level, such as preventing or reducing anemia, increasing cardiac strength, lowering blood pressure, reducing arrhythmia, making blood flow smoothly, lowering blood cholesterol, enhancing liver function, and the like.

The composition of the present invention is applicable for therapeutic use (medical use) and non-therapeutic use (non-medical use). The "non-therapeutic" is a concept that does not include medical activities, i.e., a concept that does not include methods of surgery, therapy, or diagnosis of humans.

The composition of the present invention can be provided as an agent or the like, for example, but it is not limited thereto. The agent can be directly provided as a composition or can be provided as a composition containing the agent. In one embodiment, the composition for increasing at least one of red blood cell count or hemoglobin level of the present invention can also be referred to as an agent for increasing the red blood cell count and/or hemoglobin level. In one embodiment, the composition for preventing or reducing anemia of the present invention can also be referred to as an agent for preventing or reducing anemia. In one embodiment, the composition for improving endurance of the present invention can also be referred to as an agent for improving endurance. In one embodiment, the antifatigue composition of the present invention can also be referred to as an antifatigue agent.

In order to sufficiently obtain the effect of the present invention, preferably, the composition of the present invention is an oral composition. The oral composition may be a food or beverage, an oral pharmaceutical product, an oral quasi-pharmaceutical product, or feed, preferably a food or beverage or an oral pharmaceutical product, still more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in foods, beverages, pharmaceutical products, quasi-pharmaceutical products, feed, and the like can be used. When the composition of the present invention is provided as a food or beverage, a pharmaceutical product, a quasi-pharmaceutical product, feed, or the like, the production method is not limited, and any common method can be used for production.

For example, when the composition of the present invention is provided as a food or beverage, various types of food or beverages can be provided by adding a component usable in food or beverages (e.g., a food material or an optional food additive) to L-ergothioneine or a salt thereof. Non-limiting examples of the food or beverages include general foods and beverages, health foods, health beverages, foods with function claims, foods for specified health uses, dietary supplements, and foods and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition of the present invention is not limited and can be set according to the composition form and the like.

The amount of L-ergothioneine or a salt thereof, for example, in terms of L-ergothioneine, in the composition of the present invention is preferably 0.0001 wt% or more, more preferably 0.001 wt% or more and is preferably 90 wt% or less, more preferably 50 wt% or less. In one embodiment, the amount of L-ergothioneine or a salt thereof in terms of L-ergothioneine in the composition is preferably 0.0001 to 90 wt%, more preferably 0.001 to 50 wt%. In one embodiment, when the composition of the present invention is provided as a food or beverage, a pharmaceutical product, a quasi-pharmaceutical product, feed, or the like, preferably, the amount of L-ergothioneine or a salt thereof is in the above ranges.

In the case of L-ergothioneine, the amount in terms of L-ergothioneine or an expression similar thereto refers to the amount of L-ergothioneine. In the case of a salt of L-ergothioneine, the amount in terms of L-ergothioneine or an expression similar thereto refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an effect of increasing the red blood cell count and/or hemoglobin level and may be appropriately set according to the administration form, administration method, body weight of a subject, and the like.

In one embodiment, when orally feeding or administering the composition of the present invention to a human (adult) as a subject, the intake of L-ergothioneine or a salt thereof in terms of L-ergothioneine per day is preferably 2 mg or more, more preferably 5 mg or more, still more preferably 10 mg or more, and is preferably 50 mg or less, more preferably 25 mg or less, still more preferably 20 mg or less. In one embodiment, when orally feeding or administering the composition of the present invention to a human (adult) as a subject, the intake of L-ergothioneine or a salt thereof in terms of L-ergothioneine per day is preferably 2 to 50 mg, more preferably 5 to 25 mg, still more preferably 5 to 20 mg, particularly preferably 10 to 20 mg. Preferably, the above amount of the composition is fed or administered in one or more portions per day, for example, in one portion or several portions (for example, two or three portions) per day. In one embodiment of the present invention, the composition of the present invention may be an oral composition for feeding or administering the above amount of L-ergothioneine or a salt thereof per 60 kg body weight per day to a human.

In one embodiment, when the composition of the present invention is parenterally administered to a human (adult), the administration amount of L-ergothioneine or a salt thereof in terms of L-ergothioneine per day is, for example, preferably 2 to 50 mg, more preferably 5 to 25 mg, still more preferably 5 to 20 mg, particularly preferably 10 to 20 mg.

In one embodiment, in the case of a human (adult), preferably, the above amount of L-ergothioneine or a salt thereof per 60 kg body weight per day is fed or administered.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to enhance the effect of increasing the red blood cell count and/or hemoglobin level. Thus, in a preferred embodiment, the composition of the present invention is continuously fed or administered. In one embodiment of the present invention, the composition of the present invention is continuously fed or administered preferably for at least one week, more preferably for at least two weeks.

The composition of the present invention may be fed or administered to any subject (subject to administration). The subject is preferably a human or non-human mammal, more preferably a human.

The composition of the present invention may be fed or administered to a subject needing or wanting to increase the red blood cell count and/or hemoglobin level. The subject to administration may be, for example, a human needing or wanting to prevent or reduce anemia, a human needing or wanting to improve endurance, a human needing or wanting to reduce fatigue and obtain antifatigue effect, a human needing or wanting to increase the blood taurine level, or a human needing or wanting to prevent or ameliorate a condition or disease caused by anemia. In one embodiment, the subject to administration may be, for example, a subject with anemia or a subject with a condition or disease caused by anemia. In one embodiment, the subject to administration of the composition of the present invention may be a healthy person. The composition of the present invention can be used on a healthy person, for example, for purposes such as preventing a condition or disease that is likely to be prevented or ameliorated by increasing the red blood cell count and/or hemoglobin level.

The composition of the present invention may be labeled with a function claim based on an increase in red blood cell count and/or hemoglobin level. For example, the composition of the present invention may be labeled with one or more function claims such as "increasing the red blood cell count and/or hemoglobin level", "preventing anemia", "improving endurance", "reducing fatigue", and "providing antifatigue effect".

In one embodiment of the present invention, preferably, the composition of the present invention is a food or beverage labeled with such a function claim. The label may be one indicating use of the composition for obtaining the function. The label may be directly attached to the composition or may be attached to a container or a package of the composition.

The present invention encompasses the following methods and uses:
a method of increasing at least one of red blood cell count or hemoglobin level, including feeding or administering L-ergothioneine or a salt thereof;
a method of preventing or reducing anemia, including feeding or administering L-ergothioneine or a salt thereof;
a method of improving endurance, including feeding or administering L-ergothioneine or a salt thereof;
a method of reducing, alleviating, or preventing fatigue or recovering from fatigue, including feeding or administering L-ergothioneine or a salt thereof;
a method of increasing blood taurine level, including feeding or administering L-ergothioneine or a salt thereof;
use of L-ergothioneine or a salt thereof for increasing at least one of red blood cell count or hemoglobin level;
use of L-ergothioneine or a salt thereof for preventing or reducing anemia;
use of L-ergothioneine or a salt thereof for improving endurance;
use of L-ergothioneine or a salt thereof for reducing, alleviating, or preventing fatigue or recovering from fatigue; and
use of L-ergothioneine or a salt thereof for increasing blood taurine level.

Feeding or administering L-ergothioneine or a salt thereof to a subject can increase the red blood cell count and/or hemoglobin concentration, resulting in an effect of preventing anemia, an effect of reducing anemia, an effect of improving endurance, an antifatigue effect, or the like. In one embodiment, the method of increasing at least one of red blood cell count or hemoglobin level can be used to prevent or reduce anemia, to improve endurance, or to reduce, alleviate, or prevent fatigue or recover from fatigue. Feeding or administering L-ergothioneine or a salt thereof to a subject can increase the blood taurine concentration. Preferably, L-ergothioneine or a salt thereof is orally fed or administered.

The method may be therapeutic or non-therapeutic. The use may be therapeutic or non-therapeutic.

In the methods and uses, L-ergothioneine or a salt thereof, preferred embodiments thereof, and the like are as described above for the compositions of the present invention. In the methods and uses, preferably, L-ergothioneine or a salt thereof is fed or administered to a subject one or more times per day, for example, one to several times (e.g., two to three times) per day. The uses are preferably for humans or non-human mammals, more preferably for humans. In one embodiment, L-ergothioneine or a salt thereof can be used to prevent or reduce anemia by increasing the red blood cell count and/or hemoglobin level. In one embodiment, L-ergothioneine or a salt thereof can be used to improve endurance by increasing the red blood cell count and/or hemoglobin level. In one embodiment, L-ergothioneine or a salt thereof can be used to reduce, alleviate, or prevent fatigue or recover from fatigue by increasing the red blood cell count and/or hemoglobin level. In one embodiment, L-ergothioneine or a salt thereof can be used to increase the blood taurine level. L-ergothioneine or a salt thereof is useful, for example, in promoting recovery from fatigue or the like.

In the above methods and uses, L-ergothioneine or a salt thereof is simply used in an amount (effective amount) that provides the effect of increasing the red blood cell count and/or hemoglobin level. Preferred administration amounts of L-ergothioneine or a salt thereof, preferred subjects to administration, and the like, are as described above for the composition of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can be used to produce a food or beverage, a pharmaceutical product, a quasi-pharmaceutical product, feed, or the like for use in increasing the red blood cell count and/or hemoglobin level. In one embodiment, the present invention also encompasses use of L-ergothioneine or a salt thereof for producing a composition for increasing red blood cell count and/or hemoglobin level.

The present invention also encompasses L-ergothioneine or a salt thereof for use in increasing red blood cell count and/or hemoglobin level.

L-ergothioneine or a salt thereof can also be used to produce a food or beverage, a pharmaceutical product, a quasi-pharmaceutical product, feed, or the like for use in preventing or reducing anemia, improving endurance, providing antifatigue effect, and increasing the blood taurine level. In one embodiment, the present invention also encompasses use of L-ergothioneine or a salt thereof to produce a composition for preventing or reducing anemia, a composition for improving endurance, an antifatigue composition, and a composition for increasing blood taurine level.

The present invention also encompasses L-ergothioneine or a salt thereof for use in preventing or reducing anemia, improving endurance, providing antifatigue effect, and increasing the blood taurine level.

### EXAMPLES

The present invention is described in further detail below with reference to examples, but the scope of the present invention is not limited thereto.

### <Example 1>

### (Evaluation tests on human for red blood cell count in the blood, hemoglobin concentration, and blood taurine level)

In order to evaluate the influence of supplements containing ergothioneine on the red blood cell count in the blood, blood hemoglobin concentration, and blood taurine level, a placebo-controlled, randomized, double-blind, parallel-group study was conducted on subjects including male and female adults (46 subjects in a test food group; 46 subjects in a control food group; 92 subjects in total). In this study, one capsule containing 20 mg ergothioneine (test food) or one capsule not containing ergothioneine (control food) was fed per day for four weeks. The red blood cell count in the blood, blood hemoglobin concentration, and blood taurine level of each subject were measured for pre-testing before starting the study (before starting ingestion of the test food or control food). After ingestion of the test food or control food for four weeks, the red blood cell count in the blood, blood hemoglobin concentration, and blood taurine level of each subject were measured again (testing on week 4). The red blood cell count in the blood and blood hemoglobin concentration were measured by known methods. The blood taurine level was identified by measuring blood metabolomes with a capillary electrophoresis time-of-flight mass spectrometry (CE-TOF-MS) available from Human Metabolome Technologies, Inc.

### (Food for evaluation)

Two types of food for evaluation, which were indistinguishable in appearance, flavor, and the like, were used.
- Test food: capsule containing a test substance (20 mg L-ergothioneine)
- Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

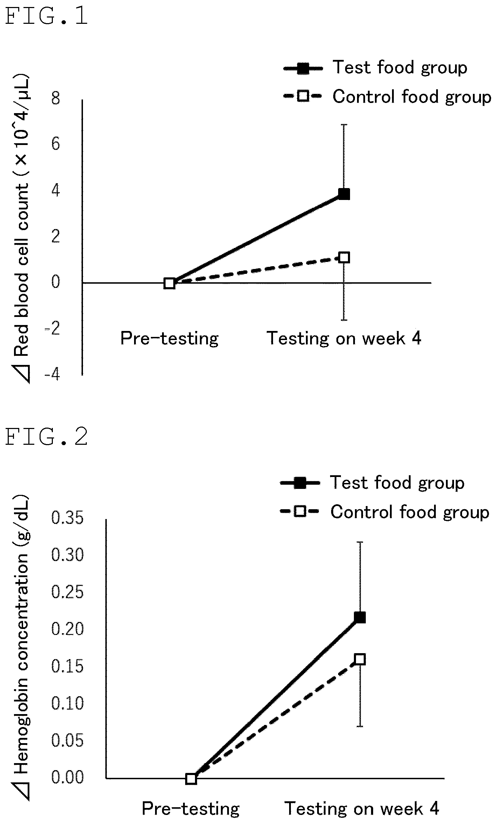

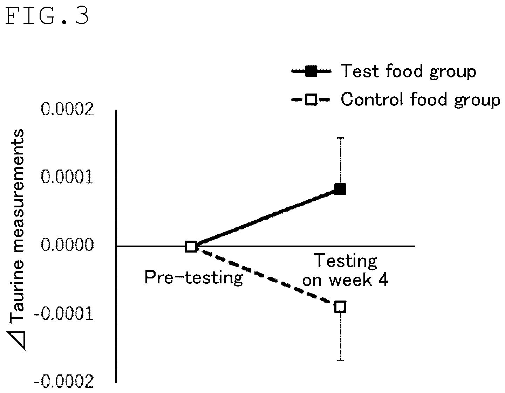

FIG. 1 and FIG. 2 each show the amount of change in red blood cell count in the blood and blood hemoglobin concentration of the test food group and the control food group. FIG. 3 shows the amount of change in blood taurine measurements of the test food group and the control food group. The amount of change shown in each of FIG. 1, FIG. 2, and FIG. 3 is the amount of change relative to the measurements at the time of pre-testing. Specifically, the amount of change in the test food group at the time of testing on week 4 was determined by subtracting the measurements (average) of the test food group at the time of pre-testing from the measurements (average) of the test food group at the time of testing on week 4. Similarly, the amount of change in the control food group at the time of testing on week 4 was determined by subtracting the measurements (average) at the time of pre-testing from the measurements (average) at the time of testing on week 4. The amount of change at pre-testing was set to 0. In FIG. 1, FIG. 2, and FIG. 3, ■ (black square) indicates the test food group, and □ (white square) indicates the control food group. In FIG. 1, "10^4" means 10⁴.

FIG. 1 is a graph showing change in red blood cell count in the blood of the test food group and the control food group between pre-testing and testing on week 4. The results show that the red blood cell count in the blood increased more significantly in the test food group than in the control food group.

FIG. 2 is a graph showing change in blood hemoglobin concentration of the test food group and the control food group between pre-testing and testing on week 4. The results show that an increase in blood hemoglobin concentration was higher in the test food group than in the control food group.

FIG. 3 is a graph showing change in blood taurine measurements of the test food group and the control food group between pre-testing and testing on week 4. The results show a significant increase in blood taurine concentration in the test food group.

## Claims

1. A composition for increasing at least one of red blood cell count or hemoglobin level, comprising
L-ergothioneine or a salt thereof as an active ingredient.

2. A composition for preventing or reducing anemia, comprising
the composition according to claim 1.

3. A composition for improving endurance, comprising
the composition according to claim 1.

4. An antifatigue composition, comprising
the composition according to claim 1.

5. The composition according to any one of claims 1 to 4 for increasing blood taurine level.

6. The composition according to any one of claims 1 to 5,
wherein the composition is an oral composition.

7. The composition according to any one of claims 1 to 6,
wherein the composition is a food or beverage.

8. The composition according to any one of claims 1 to 7,
wherein an amount of L-ergothioneine or a salt thereof in the composition per adult daily intake is 2 to 50 mg in terms of L-ergothioneine.

9. A method of increasing at least one of red blood cell count or hemoglobin level, comprising
administering L-ergothioneine or a salt thereof.

10. A method of preventing or reducing anemia, comprising
administering L-ergothioneine or a salt thereof.

11. A method of improving endurance, comprising administering L-ergothioneine or a salt thereof.

12. A method of reducing, alleviating, or preventing fatigue or recovering from fatigue, comprising
administering L-ergothioneine or a salt thereof.

13. Use of L-ergothioneine or a salt thereof for increasing at least one of red blood cell count or hemoglobin level.

14. Use of L-ergothioneine or a salt thereof for preventing or reducing anemia.

15. Use of L-ergothioneine or a salt thereof for improving endurance.

16. Use of L-ergothioneine or a salt thereof for reducing, alleviating, or preventing fatigue or recovering from fatigue.
